# EUROPÄISCHE PATENTSCHRIFT

(11) **EP 1 021 431 B1**
(45) Veröffentlichungstag und Bekanntmachung des Hinweises auf die Patenterteilung: **02.05.2002**
(21) Anmeldenummer: 98951481.5
(22) Anmeldetag: 30.09.1998
(51) Int. Cl.: C07D 307/83

(54) **VERFAHREN ZUR HERSTELLUNG GEGEBENENFALLS SUBSTITUIERTER BENZOFURANONE**
METHOD FOR PREPARING OPTIONALLY SUBSTITUTED BENZOFURANONES
PROCEDE DE PREPARATION DE BENZOFURANONES EVENTUELLEMENT SUBSTITUEES

(30) Priorität: 10.10.1997 DE 19744705
(43) Veröffentlichungstag der Anmeldung: 26.07.2000
(73) Patentinhaber: BAYER AG, 51368 Leverkusen (DE)
(72) Erfinder: GALLENKAMP, Bernd, D-42113 Wuppertal (DE); GERDES, Peter, D-52080 Aachen (DE); GAYER, Herbert, D-40789 Monheim (DE); ROHE, Lothar, D-42113 Wuppertal (DE); MULDER, Lubbertus, D-58135 Hagen (DE)
(86) Internationale Anmeldenummer: EP9806213
(87) Internationale Veröffentlichungsnummer: WO9919316

(56) Entgegenhaltungen:
- D.C. SCHROEDER ET AL.: "Benzofuro(3,2-b)indoles" JOURNAL OF ORGANIC CHEMISTRY., Bd. 27, Nr. 2, 1962, Seiten 586-591, XP002093643 EASTON US in der Anmeldung erwähnt
- CHEMICAL ABSTRACTS, vol. 83, no. 9, 1. September 1975 Columbus, Ohio, US; abstract no. 79112, K. GOERLITZER: "1,3-Dicarbonyl compounds" XP002093644 & ARCH. PHARM., Bd. 308, Nr. 4, 1975, Seiten 272-286,
- CHEMICAL ABSTRACTS, vol. 86, no. 15, 11. April 1977 Columbus, Ohio, US; abstract no. 106264, S.B. MAHAYAN ET AL.: "A convenient method for the synthesis of 2-carbethoxy-3-(2H)-benzofuranone" XP002093645 & CURR. SCI., Bd. 45, Nr. 20, 1976, Seite 722

## Beschreibung

Die Erfindung betrifft ein verbessertes Verfahren zur Herstellung von gegebenenfalls substituierten Benzofuran-3-onen, die als Vorprodukte zur Herstellung von 3-(1-Hydroxyphenyl-1-alkoximinomethyl)dioxazinen eingesetzt werden können, die wiederum als Ausgangsstoffe zur Herstellung von Verbindungen mit fungiziden Eigenschaften bekannt sind (WO 95-04728 und DE-A 9602095).

Es ist bereits bekannt geworden, daß gegebenenfalls substituierte Benzofuran-3-one gemäß nachfolgendem Reaktionsschema hergestellt werden können (J. Org. Chem. 1962, 586-591):

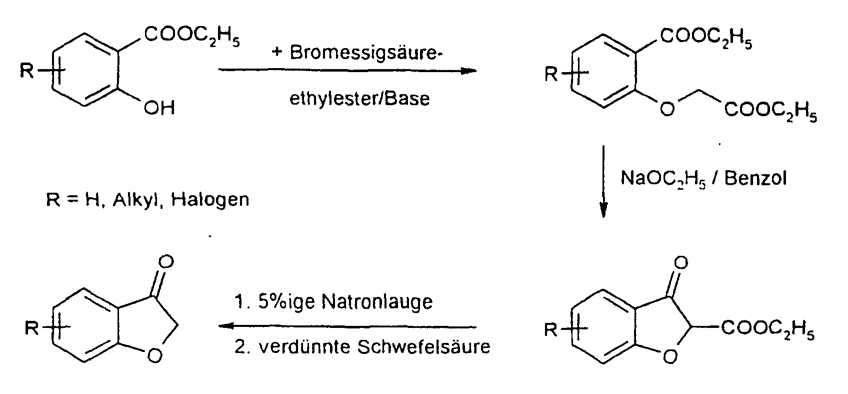

Zunächst wird ein Salicylsäureethylester mit Bromessigsäureethylester umgesetzt und der so erhaltene Salicylsäureether mit Natriumethanolat in Benzol zu einem Benzofuranoncarbonsäureethylester cyclisiert. Dieser wird in verdünnter Natronlauge hydrolysiert und anschließend mit verdünnter Schwefelsäure decarboxyliert und ausgefällt. Die Ausbeuten dieses Verfahrens sind äußerst unbefriedigend und die Reaktionszeiten, insbesondere die Hydrolyse dauert eine Woche oder länger, sind für eine technische Herstellung nicht akzeptabel.

Eine alternative Synthese zur Herstellung von gegebenenfalls substituierten Benzofuranonen wird in Synthetic Communications 1990, 809-816 beschrieben:

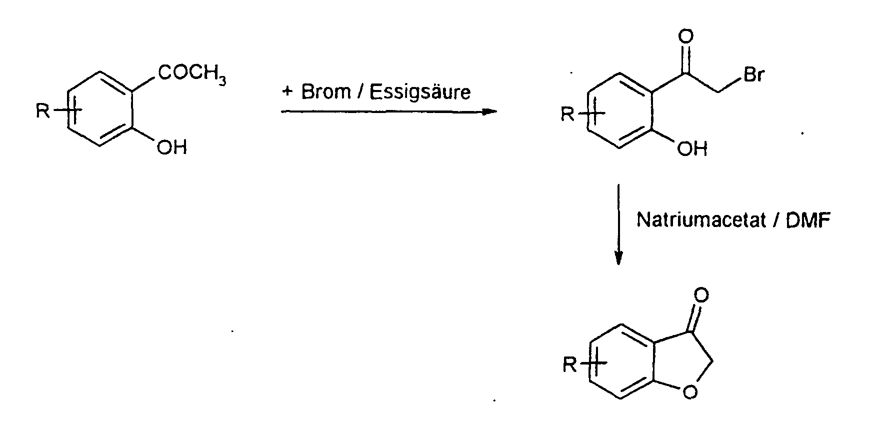

Hierbei wird zuerst o-Hydroxyacetophenon mit Brom in Essigsäure bromiert und die bromierte Verbindung mit Natriumacetat in Dimethylformamid unter Bromwasserstoffabspaltung cyclisiert. Auch bei diesem Verfahren sind die Ausbeuten unbefriedigend. Außerdem ließe sich das als Reagenz benötigte Brom nur mit hohem Sicherheitsaufwand in einer technischen Synthese einsetzen.

Es wurde nun gefunden, daß man gegebenenfalls substituierte Benzofuranone der Formel (I) 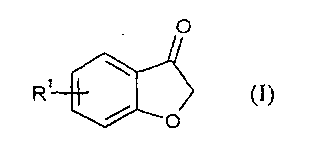 in welcher
- R¹: für Wasserstoff, Alkyl oder Halogen steht,
in hoher Ausbeute und hoher Reinheit erhält, wenn man Salicylsäureether der allgemeinen Formel (II) 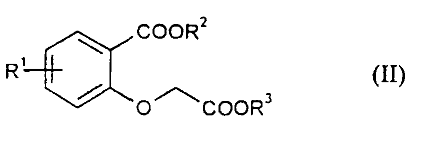 in welcher
- R¹: die oben angegebene Bedeutung hat und
- R² und R³: gleich oder verschieden sind und unabhängig voneinander für Alkyl stehen,
in einem ersten Schritt mit einer starken Base, vorzugsweise einem Alkalimetallalkoholat, unter einer Schutzgasatmosphäre, gegebenenfalls in Gegenwart eines Verdünnungsmittels, umsetzt und den auf diese Weise erhaltenen Benzofuranoncarbonsäureester der Formel (III) 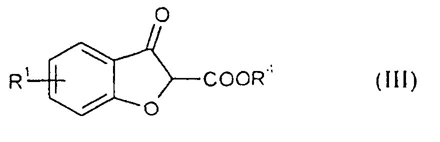 in welcher
- R¹: die oben angegebene Bedeutung hat und
- R⁴: für Alkyl steht,
in einem zweiten Schritt zuerst mit Kaliumhydroxid gegebenenfalls in Gegenwart eines Verdünnungsmittels und anschließend mit einer Säure zu den gewünschten Benzofuranonen der Formel (I) umsetzt.

Bevorzugt herstellbar sind Benzofuranone der Formel (I), in welcher
- R¹: für Wasserstoff, Alkyl mit 1 bis 4 Kohlenstoffatomen, Fluor oder Chlor steht.

Besonders bevorzugt herstellbar sind Benzofuranone der Formel (I), in welcher
- R¹: für Wasserstoff, Methyl, Ethyl, Fluor oder Chlor steht.

Bevorzugt herstellbare Zwischenprodukte der Formel (III) sind diejenigen Benzofuranoncarbonsäureester der Formel (III), in welcher
- R¹: für Wasserstoff, Alkyl mit 1 bis 4 Kohlenstoffatomen, Fluor oder Chlor steht und
- R⁴: für Alkyl mit 1 bis 4 Kohlenstoffatomen steht.

Besonders bevorzugt herstellbare Zwischenprodukte der Formel (III) sind diejenigen Benzofuranoncarbonsäureester der Formel (III), in welcher
- R¹: für Wasserstoff. Methyl, Ethyl, Fluor oder Chlor steht und
- R⁴: für Methyl oder Ethyl steht.

Es ist als ausgesprochen überraschend zu bezeichnen, daß der erste Schritt des erfindungsgemäßen Verfahrens durch das Arbeiten unter den erfindungsgemäßen Bedingungen zu geringerer Nebenproduktbildung neigt und so wesentlich höhere Ausbeuten liefert als das nach dem Stand der Technik bekannte Verfahren. Es ist weiterhin als besonders überrachend anzusehen, daß der zweite Schritt des erfindungsgemäßen Verfahrens durch die Anwendung der erfindungsgemäßen Reaktionsbedingungen wesentlich schneller und mit erheblich höheren Ausbeuten und Reinheiten verläuft als bereits beschriebene Verfahren.

Das erfindungsgemäße Verfahren zeichnet sich durch eine Reihe von Vorteilen aus. So ermöglicht es, die Synthese von Benzofuranonen der Formel (I) in sehr hoher Ausbeute und Reinheit. Günstig ist auch, daß die benötigten Reaktionskomponenten einfach herstellbar und auch in größeren Mengen verfügbar sind. Ein weiterer Vorteil besteht schließlich darin, daß die Durchführung der Umsetzung und die Isolierung der Reaktionsprodukte keinerlei Schwierigkeiten bereitet

Die zur Durchführung der ersten Stufe des erfindungsgemäßen Verfahrens als Ausgangsstoffe benötigten Salicylsäureether sind durch die allgemeine Formel (II) definiert. In dieser Formel (II) hat R¹ vorzugsweise bzw. insbesondere diejenige Bedeutung, die bereits im Zusammenhang mit der Beschreibung der erfindungsgemäß herstellbaren Verbindungen der Formel (I) als bevorzugt bzw. als insbesondere bevorzugt für R¹ angegeben wurde. R² und R³ stehen unabhängig voneinander für Alkyl, vorzugsweise mit 1 bis 4 Kohlenstoffatomen, insbesondere für Ethyl oder Methyl.

Die Salicylsäureether der Formel (II) sind bekannt und/oder nach bekannten Methoden herstellbar (vergleiche z. B. J. Chem. Soc. 99 (1911), 911; und die Herstellungsbeispiele).

Als Verdünnungsmittel zur Durchführung der ersten Stufe des erfindungsgernäßen Verfahrens kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Amide, wie N,N-Dimethylformamid, N,N-Dimethylacetamid, N-Methylformanilid, N-Methylpyrrolidon oder Hexamethylphosphorsäuretriamid; Alkohole, wie Methanol, Ethanol, n- oder i-Propanol, n-, i-, sekoder tert-Butanol, Ethandiol, Propan-1,2-diol, Ethoxyethanol, Methoxyethanol, Diethylenglykolmonomethylether, Diethylenglykolmonoethylether, sowie beliebige Gemische der genannten Verdünnungsmittel.

Die erste Stufe des erfindungsgemäßen Verfahrens wird in Gegenwart einer Base durchgeführt. Als solche kommen vorzugsweise Erdalkalimetall- oder Alkalimetallhydride oder -alkoholate, wie beispielsweise Natriumhydrid, Natriummethylat, Natriumethylat, Kalium-tert.-butylat infrage.

Die erste Stufe des erfindungsgemäßen Verfahrens wird vorzugsweise unter einer Schutzgasatmosphäre durchgeführt. Als Schutzgase kommen beispielsweise Stickstoff oder Argon, aber auch Lösungsmitteldämpfe infrage.

Die Reaktionstemperaturen können bei der Durchführung der ersten Stufe des erfindungsgemäßen Verfahrens in einem größeren Bereich variiert werden. Im allgemeinen arbeitet man bei Temperaturen von 0°C bis 150°C, vorzugsweise bei Temperaturen von 0°C bis 80°C.

Zur Durchführung der ersten Stufe des erfindungsgemäßen Verfahrens zur Herstellung der Verbindungen der Formel (I) setzt man pro Mol des Salicylsäureethers der Formel (II) im allgemeinen 0,5 bis 10 Mol, vorzugsweise 1 bis 4 Mol Base ein.

Bei der Durchführung der ersten Stufe des erfindungsgemäßen Verfahrens geht man im allgemeinen wie folgt vor: Der Salicylsäureether der Formel (II) wird in einem Verdünnungsmittel gelöst, bzw. suspendiert und dann erwärmt. Hierzu gibt man langsam die Base, die gegebenenfalls in einem Verdünnungsmittel vorgelöst ist. Nach dem Ende der Reaktion wird nach üblichen Methoden aufgearbeitet. Beispielsweise wird die Reaktionsmischung in Wasser gegeben, die gegebenenfalls vorhandene organische Phase abgetrennt und verworfen, die wäßrige Phase angesäuert, das kristallisierte Produkt abgesaugt und gegebenenfalls getrocknet.

Während der gesamten Durchführung der Reaktion und der Aufarbeitung wird Luft-bzw. Sauerstoffzutritt zum Produkt vermieden. Dies geschieht, indem man entweder unter einer Schutzgasatmosphäre, beispielsweise unter Stickstoff oder Argon, arbeitet, oder die Reaktionsmischungen sorgfältig, beispielsweise durch die darüberliegenden Lösungsmitteldämpfe, von der Außenluft abschirmt. Beispielsweise wird durch das Erwärmen der Lösung des Ausgangsstoffes der Formel (II) gewährleistet, daß sich keine Luft, sondern nur Lösungsmitteldampf unmittelbar über der Oberfläche der Lösung befindet. Damit wird zuverlässig verhindert, daß das Produkt, das nach Zugabe der Base entsteht, mit Sauerstoff in Berührung kommt. Die gegebenenfalls erwünschte Trocknung des Produktes erfolgt im Vakuum; die Aufbewahrung des Produktes erfolgt in einem geschlossenen Gefäß unter einer Schutzgasatmosphäre, beispielsweise unter Stickstoff oder Argon.

Verwendet man als Verdünnungsmittel bzw. Verdünnungsmittelkomponente einen Alkohol, der verschieden ist von R³-OH, bzw. als Base ein Alkoholat, dessen Alkylgruppe von R³ verschieden ist, so erfolgt neben dem erwünschten Ringschluß gegebenenfalls auch eine teilweise oder vollständige Umesterung der Estergruppe des entstehenden Benzofuranoncarbonsäureesters der Formel (III). Für die Durchführung der zweiten Stufe des erfindungsgemäßen Verfahrens ist dies jedoch unerheblich.

Als Verdünnungsmittel zur Durchführung der zweiten Stufe des erfindungsgemäßen Verfahrens kommen alle inerten organischen Lösungsmittel in Betracht. Hierzu gehören vorzugsweise Ether, wie Diethylether, Diisopropylether, Methyl-t-butylether, Methyl-t-Amylether, Dioxan, Tetrahydrofuran, 1,2- Dimethoxyethan, 1,2-Diethoxyethän oder Anisol; Amide, wie N,N-Dimethylformamid, N,N-Dimethylacetamid, N-Methylformanilid, N-Methylpyrrolidon oder Hexamethylphosphorsäuretriamid; Sulfone, wie Sulfolan; Alkohole, wie Methanol, Ethanol, n- oder i-Propanol, n-, i-, sek-oder tert-Butanol, Ethandiol, Propan-1,2-diol, Ethoxyethanol, Methoxyethanol, Diethylenglykolinonomethylether, Diethylenglykolmonoethylether, deren Gemische untereinander, sowie deren Gemische mit Wasser oder reines Wasser.

Zur Durchführung der zweiten Stufe des erfindungsgemaßen Verfahrens wird eine Säure benötigt. Als solche kommen alle anorganischen und organischen Protonensäuren infrage. Vorzugsweise verwendbar sind Chlorwasserstoffsäure, Schwefelsäure oder Phosphorsäure.

Die zweite Stufe des erfindungsgemäßen Verfahrens wird vorzugsweise unter einer Schutzgasatmosphäre durchgeführt. Als Schutzgase kommen beispielsweise Stickstoff oder Argon infrage.

Die Reaktionstemperaturen können bei der Durchfuhrung der zweiten Stufe des erfindungsgemäßen Verfahrens in einem größeren Bereich variiert werden. Im allgemeinen arbeitet man bei Temperaturen von 0°C bis 150°C, vorzugsweise bei Temperaturen von 0°C bis 80°C.

Zur Durchführung der zweiten Stufe des erfindungsgemäßen Verfahrens zur Herstellung der Verbindungen der Formel (I) setzt man pro Mol des Benzofuranoncarbonsäureesters der Formel (III) im allgemeinen 1 bis 10 Mol, vorzugsweise 2 bis 8 Mol Kaliumhydroxid ein.

Beide Stufen des erfindungsgemäßen Verfahrens werden im allgemeinen unter Normaldruck durchgefuhrt. Es ist jedoch auch möglich, unter erhöhtem oder vermindertem Druck - im allgemeinen zwischen 0,1 bar und 10 bar - zu arbeiten.

Bei der Durchführung der zweiten Stufe des erfindungsgemäßen Verfahrens geht man im allgemeinen wie folgt vor: Zu einer Kaliumhydroxidlösung gibt man den in der ersten Stufe des erfindungsgemäßen Verfahrens erhaltenen Benzofuranoncarbonsäureester der Formel (II) zu und erwärmt die Reaktionsmischung. Nach dem Ende der Reaktion wird die Reaktionsmischung gegebenenfalls verdünnt und dann mit einer Säure versetzt oder vorzugsweise wird die Reaktionsmischung gegebenenfalls verdünnt und dann in eine Säure hineingetropft. Die Aufarbeitung erfolgt nach üblichen Methoden. Beispielsweise wird das Produkt, das nach dem Abkühlen aus der Reaktionsmischung ausfällt, abgesaugt und gegebenenfalls weiter, beispielsweise durch Wasserdampfdestillation, aufgereinigt. Während der gesamten Durchführung der Reaktion und der Aufarbeitung ist es von Vorteil, Luft- bzw. Sauerstoffzutritt zum Produkt zu vermeiden.

### Herstellungsbeispiele:

### Beispiel 1

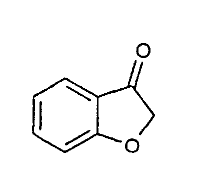

### 1. Stufe:

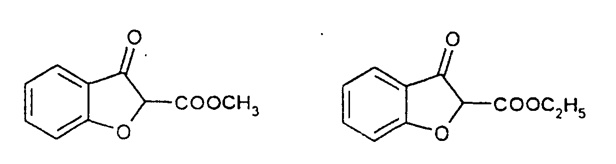

8200 g (32,25 Mol) 2-Ethoxycarbonylmethoxybenzoesäuremethylester wird in 26 l Toluol suspendiert und auf 50°C erwärmt. Bei dieser Temperatur tropft man innerhalb von 15 Minuten 6383 g (35,46 Mol) Natriummethanolatlösung (30 % in Methanol) zu und rührt 2 Stunden bei 50°C. Das Reaktionsgemisch wird auf 20°C abgekühlt und in 82 l Eiswasser gegeben, wobei sich zwei Phasen ausbilden. Die organische Phase wird abgetrennt und verworfen. Die wäßrige Phase wird mit 10 l Toluol gewaschen, filtriert und bei 15 - 20°C mit konzentrierter Salzsäure auf einen pH-Wert von 1 - 2 eingestellt. Die dabei entstandene Suspension wird mit 16 l Wasser verdünnt, gerührt und abgesaugt. Das Produkt wird mit 13 l Wasser portionsweise nachgewaschen und feucht unter Stickstoffatmosphäre aufbewahrt. Man erhält 9500 g wasserfeuchtes Produkt (5063,5 g = 53,3 % Trockensubstanz). Die Trockensubstanz enthält 85,64 % Benzofuran-3-on-2-carbonsäuremethylester (HPLC; logP = 1,88) und 14,36 % Benzofuran-3-on-2-carbonsäureethylester (HPLC; logP = 2,32). Die Gesamtausbeute beträgt 84 %. Während der gesamten Durchführung der Reaktion und der Aufarbeitung wird Luft- bzw. Sauerstoffzutritt zum Produkt vermieden.

### 2. Stufe

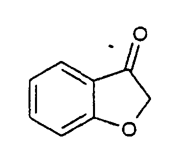

Man bereitet eine Lösung aus 8,2 l Wasser und 8262 g (125,4 Mol) Kaliumhydroxid (85 %), gibt bei 50 - 60°C 12,5 l Ethanol zu und anschließend 9079 g des in der oben beschriebenen 1. Stufe erhaltenen wasserfeuchten Gemisches aus Benzofuran-3-on-2-carbonsäuremethylester und Benzofuran-3-on-2-carbonsäureethylester (insgesamt 24,79 Mol). Es wird 45 Minuten unter Rückfluß erhitzt und danach 7,3 l Wasser zugegeben. Diese Lösung wird auf 20°C abgekühlt und innerhalb von 60 Minuten unter heftiger Gasentwicklung in eine auf 55°C erwärmte Mischung aus 17,5 l konzentrierter Salzsäure und 20 l Wasser gegeben. Reste der alkalischen Lösung werden mit 3 l Wasser ausgespült und ebenfalls in die Salzsäure gegeben. es wird noch 30 Minuten bei 60°C gerührt (Ende der Gasentwicklung) und auf 20°C abgekühlt. Das Produkt wird abgesaugt und mit 6 l Wasser nachgewaschen und im Vakuumtrockenschrank bei 35°C getrocknet. Man erhält 3250 g (94 % der Theorie) Benzofuran-3-on. HPLC: logP = 1,26

### Herstellung des Ausgangsstoffes:

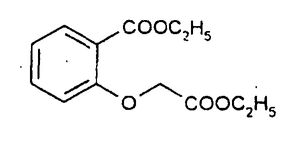

Zu einer Mischung aus 62 l Acetonitril, 9490 g (62,38 Mol) Salicylsäuremethylester 8610 g (62,38 Mol) Kaliumcarbonat und 620 g Kaliumiodid gibt man bei 20°C 8020 g (65,44 Mol) Chloressigsäureethylester, rührt und erhitzt 9 Stunden unter Rückfluß und rührt über Nacht ohne weitere Wärmezufuhr. Die Mischung wird über Celite filtriert und das Filtrat wird bei vermindertem Druck eingeengt. Man erhält 16,4 kg 2-Ethoxycarbonylmethoxybenzoesäuremethylester (HPLC: logP = 2,1)

### Verwendungsbeispiel:

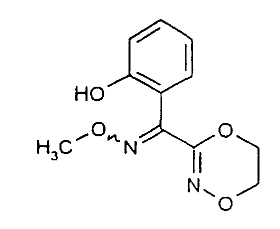

### 1. Stufe:

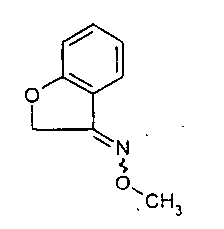

6,7 g (0,05 Mol) Benzofuran-3-on werden mit 4,2 g (0,05 Mol) O-Methylhydroxylamin-Hydrochlorid und 4,1 g (0,05 Mol) Natriumacetat in 50 ml Methanol 3 Stunden unter Rückfluß gekocht. Man destilliert das Lösungsmittel im Vakuum ab, gießt das Reaktionsgemisch auf Wasser und extrahiert mit Essigsäureethylester. Man wäscht die organische Phase mit gesättigter, wäßriger Natriumcarbonatlösung. Man trocknet die organische Phase über Natriumsulfat und destilliert das Lösungsmittel im Vakuum ab. Man erhält 7,27 g (89,2 % der Theorie) rohes Benzofuran-3-on-O-methyl-oxim. Zur Analyse destilliert man es bei 2 Torr und 70°C im Kugelrohr. Man erhält ein Öl, das sowohl entsprechend der NMR-Analyse als auch entsprechend der HPLC-Analyse aus zwei Stereoisomeren besteht (79 % Isomer B und 21 % Isomer A).

### 2. Stufe

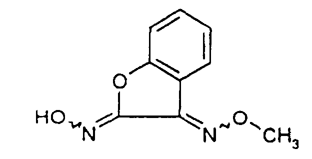

Zu 30 ml mit trockenem Chlorwasserstoff gesättigtem Essigsäureethylester tropft man bei -10°C 2 g (0,019 Mol) tert.-Butylnitrit und rührt 15 Minuten bei dieser Temperatur. Dann gibt man bei -10°C 1,6 g (0,0098 Mol) Benzofuran-3-on-O-methyl-oxim, gelöst in 5 ml Essigsäureethylester zu, läßt die Temperatur auf 0°C steigen und rührt 30 Minuten bei dieser Temperatur. Man filtriert das auskristallisierte Produkt ab und erhält 1,08 g kristallines Benzofuran-2,3-dion-3-(O-methyl-oxim)-2-oxim als Gemisch von zwei Stereoisomeren, das entsprechend der HPLC-Analyse zu 54,7 % (56 % der Theorie) aus Isomer B und zu 42,9 % aus Isomer A besteht.
¹H-NMR-Spektrum (CDCl₃/TMS): δ = 4,10 (3H, Isomer B); 4,11 (3H, Isomer A); 7,21-7,26 (1H); 7,31-7,35 (1H); 7,5-7,65 (2H, Isomer B + 1H, Isomer A); 8,02-8,05 (1H, Isomer A); 11,36 (1H, Isomer A); 11,75 (1H, Isomer B) ppm.

### 3 Stufe:

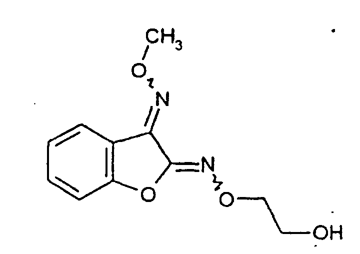

In eine Lösung von 192,2 g (1,0 Mol) Benzofuran-2,3-dion-3-(O-methyl-oxim)-2-oxim in 2 l Wasser leitet man innerhalb von 85 Minuten bei 20°C 264,3 g (6,0 Mol) Ethylenoxid ein. Die Lösung wird auf 5°C gekühlt und 70 g (1,06 Mol) Kaliumhydroxidplätzchen zugegeben, wobei die Temperatur auf 10°C ansteigt. Es wird noch 165 Minuten ohne weitere Kühlung gerührt, der entstandene Niederschlag abgesaugt, portionsweise mit 500 ml Eiswasser gewaschen und im Vakuumtrockenschrank bei 40°C getrocknet. Man erhält 143,0 g (61 % der Theorie) Benzofuran-2,3-dion-2-[O-(2-hydroxy-ethyl)-oxim]-3-(O-methyl-oxim) als Gemisch von zwei Stereoisomeren.
HPLC: logP = 1,65 (0,5 %); 1,79 (99,5 %)

### 4. Stufe

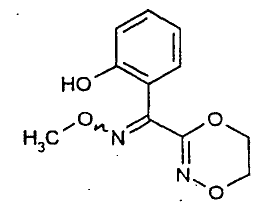

Eine Lösung aus 25,6 g (0,1084 Mol) Benzofuran-2,3-dion-2-[O-(2-hydroxy-ethyl)-oxim]-3-(O-methyl-oxim) und 14,2 g (0,216 Mol) Kaliumhydroxid-Plätzchen in 250 ml Wasser wird 195 Minuten bei 60°C gerührt. Man kühlt die Lösung auf 10°C ab und säuert mit mit Eissessig auf einen pH-Wert von 5 - 6 an. Das auskristallisierte Produkt wird abgesaugt, portionsweise mit 200 ml Wasser gewaschen und bei 45°C im Vakuumtrockenschrank getrocknet. Man erhält 17,7g (67,7 % der Theorie) E-(5,6-Dihydro-[1,4,2]dioxazin-3-yl)-(2-hydroxy-phenyl)-methanon-O-methyl-oxim.
HPLC: logP= 1,22

Die Bestimmung der logP-Werte erfolgte gemäß EEC-Directive 79/831 Annex V. A8 durch HPLC (Gradientenmethode, Acetonitril/0,1% wäßrige Phosphorsäure)

## Patentansprüche

1. Verfahren zur Herstellung von Verbindungen der Formel (I) 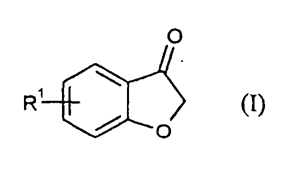 in welcher
R¹ für Wasserstoff, Alkyl oder Halogen steht,
**dadurch gekennzeichnet, daß** man Salicylsäureether der allgemeinen Formel (II) 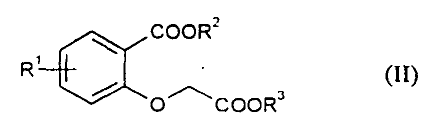 in welcher
R¹ die oben angegebene Bedeutung hat und
R² und R³ gleich oder verschieden sind und unabhängig voneinander für Alkyl stehen,
in einem ersten Schritt mit einer starken Base, gegebenenfalls unter einer Schutzgasatmosphäre und/oder in Gegenwart eines Verdünnungsmittels, umsetzt und den auf diese Weise erhaltenen Benzofuranoncarbonsäureester der Formel (III) 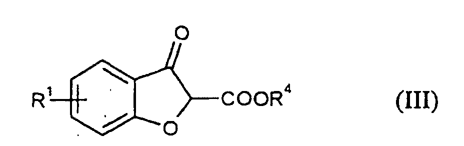 in welcher
R¹ die oben angegebene Bedeutung hat und
R⁴ für Alkyl steht,
in einem zweiten Schritt zuerst mit Kaliumhydroxid gegebenenfalls in Gegenwart eines Verdünnungsmittels und anschließend mit einer Säure zu den gewünschten Benzofuranonen der Formel (I) umsetzt.

2. Verfahren gemäß Anspruch 1, **dadurch gekennzeichnet, daß** Verbindungen der Formel (I), in welcher
R¹ für Wasserstoff, Alkyl mit 1 bis 4 Kohlenstoffatomen, Fluor oder Chlor steht,
hergestellt werden.

3. Verfahren gemäß Anspruch 1, **dadurch gekennzeichnet, daß** Verbindungen der Formel (I), in welcher
R¹ für Wasserstoff, Methyl, Ethyl, Fluor oder Chlor steht, hergestellt werden.

4. Verfahren gemäß Anspruch 1, **dadurch gekennzeichnet, daß** als Base Erdalkalimetall- oder Alkalimetallhydride oder -alkoholate eingesetzt werden.

5. Verfahren gemäß Anspruch 1, **dadurch gekennzeichnet, daß** das Verfahren in der ersten Stufe bei Temperaturen von 0°C bis 150°C durchgeführt wird.

6. Verfahren gemäß Anspruch 1, **dadurch gekennzeichnet, daß** das Verfahren in der zweiten Stufe bei Temperaturen von 0°C bis 150°C durchgeführt wird.

7. Verfahren gemäß Anspruch 1, **dadurch gekennzeichnet, daß** man pro Mol des Salicylsäureethers der Formel (II) im allgemeinen 0,5 bis 10 Mol Base einsetzt.

8. Verfahren gemäß Anspruch 1, **dadurch gekennzeichnet, daß** in der zweiten Stufe des Verfahrens Chlorwasserstoffsäure, Schwefelsäure oder Phosphorsäure eingesetzt werden.

9. Verfahren gemäß Anspruch 1, **dadurch gekennzeichnet, daß** man pro Mol Benzofuranoncarbonsäureester der Formel (III) im allgemeinen 1 bis 10 Mol Kaliumhydroxid einsetzt.

## Claims

1. Process for the preparation of compounds of the formula (I) 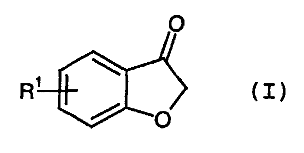 in which
R¹ represents hydrogen, alkyl or halogen,
**characterized in that** salicylic acid ethers of the general formula (II) 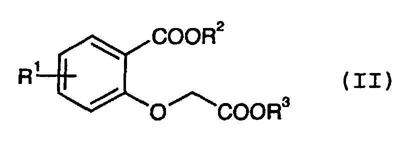 in which
R¹ has the abovementioned meaning and
R² and R³ are identical or different and independently of one another represent alkyl,
are reacted in a first step with a strong base, optionally under an inert gas atmosphere and/or in the presence of a diluent, and the benzofuranonecarboxylic acid ester obtained in this manner, of the formula (III) 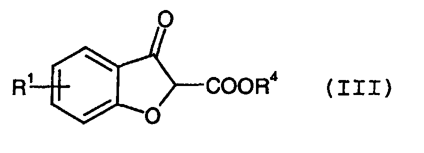 in which
R¹ has the abovementioned meaning and
R⁴ represents alkyl,
is reacted in a second step first with potassium hydroxide, optionally in the presence of a diluent, and then with an acid to give the desired benzofuranones of the formula (I).

2. Process according to Claim 1, **characterized in that** compounds of the formula (I) in which
R¹ represents hydrogen, alkyl having 1 to 4 carbon atoms, fluorine or chlorine
are prepared.

3. Process according to Claim 1, **characterized in that** compounds of the formula (I) in which
R¹ represents hydrogen, methyl, ethyl, fluorine or chlorine
are prepared.

4. Process according to Claim 1, **characterized in that** alkaline earth metal or alkali metal hydrides or alcoholates are employed as the base.

5. Process according to Claim 1, **characterized in that** the process in the first stage is carried out at temperatures from 0°C to 150°C.

6. Process according to Claim 1, **characterized in that** the process in the second stage is carried out at temperatures from 0°C to 150°C.

7. Process according to Claim 1, **characterized in that** in general 0.5 to 10 mol of base are employed per mole of the salicylic acid ether of the formula (II).

8. Process according to Claim 1, **characterized in that** hydrochloric acid, sulphuric acid or phosphoric acid are employed in the second stage of the process.

9. Process according to Claim 1, **characterized in that** in general 1 to 10 mol of potassium hydroxide are employed per mole of benzofuranonecarboxylic acid ester of the formula (III).

## Revendications

1. Procédé de production de composés de formule (I) 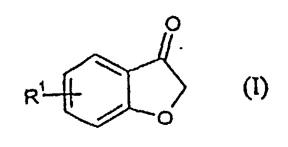 dans laquelle
R¹ représente l'hydrogène, un groupe alkyle ou un halogène,
**caractérisé en ce qu'**on fait réagir un éther d'acide salicylique de formule générale (II) 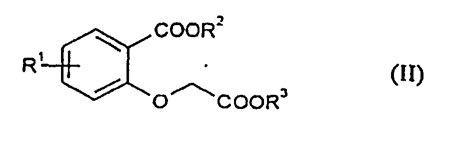 dans laquelle
R¹ a la définition indiquée ci-dessus et
R² et R³ sont identiques ou différents et représentent, indépendamment l'un de l'autre, un groupe alkyle,
dans une première étape avec une base forte, éventuellement sous une atmosphère de gaz protecteur et/ou en présence d'un diluant, et on fait réagir dans une seconde étape l'ester d'acide benzofurannone-carboxylique ainsi obtenu de formule (III) 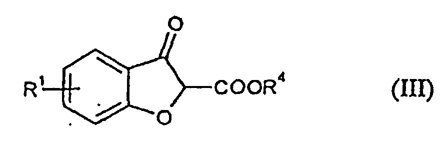 dans laquelle
R¹ a la définition indiquée ci-dessus et
R⁴ est un groupe alkyle,
tout d'abord avec l'hydroxyde de potassium, le cas échéant en présence d'un diluant, puis avec un acide pour former les benzofurannones désirées de formule (I).

2. Procédé suivant la revendication 1, **caractérisé en ce que** des composés de formule (I) dans laquelle
R¹ représente l'hydrogène, un groupe alkyle ayant 1 à 4 atomes de carbone, le fluor ou le chlore,
sont produits.

3. Procédé suivant la revendication 1, **caractérisé en ce que** des composés de formule (I) dans laquelle
R¹ représente l'hydrogène, le groupe méthyle, éthyle, le fluor ou le chlore, sont produits.

4. Procédé suivant la revendication 1, **caractérisé en ce qu'**on utilise comme base des hydrures ou des alcoolates de métaux alcalino-terreux ou de métaux alcalins.

5. Procédé suivant la revendication 1, **caractérisé en ce qu'**il est mis en oeuvre dans la première étape à des températures de 0°C à 150°C.

6. Procédé suivant la revendication 1, **caractérisé en ce qu'**il est mis en oeuvre dans la seconde étape à des températures de 0°C à 150°C.

7. Procédé suivant la revendication 1, **caractérisé en ce qu'**on utilise généralement 0,5 à 10 moles de base par mole de l'éther d'acide salicylique de formule (II).

8. Procédé suivant la revendication 1, **caractérisé en ce qu'**on utilise de l'acide chlorhydrique, de l'acide sulfurique ou de l'acide phosphorique dans la seconde étape du procédé.

9. Procédé suivant la revendication 1, **caractérisé en ce qu'**on utilise généralement 1 à 10 moles d'hydroxyde de potassium par mole d'ester d'acide benzofurannone-carboxylique de formule (III).
